(19) Europäisches Patentamt
European Patent Office
Office européen des brevets

(11) Publication number: **0 021 841 B1**

(12) **EUROPEAN PATENT SPECIFICATION**

(45) Date of publication of patent specification: **24.10.84**

(21) Application number: **80302171.6**

(22) Date of filing: **27.06.80**

(51) Int. Cl.³: **C 07 C 50/28,** C 07 C 66/00, C 07 C 69/738, C 07 C 69/00, C 07 C 46/02, C 07 C 46/08, C 07 C 51/373, C 07 C 67/39, C 07 C 67/29

# (54) 2,3-Dialkoxy-1,4-quinone derivatives; method of producing 1,4-quinone derivatives.

(30) Priority: **28.06.79 JP 82381/79**
**15.04.80 JP 49956/80**

(43) Date of publication of application:
**07.01.81 Bulletin 81/01**

(45) Publication of the grant of the patent:
**24.10.84 Bulletin 84/43**

(84) Designated Contracting States:
**BE DE FR GB IT**

(56) References cited:
**EP-A-0 031 727**
**FR-A-2 268 778**
**FR-A-2 269 333**
**FR-A-2 272 068**
**FR-A-2 293 194**
**US-A-3 959 317**
**US-A-3 966 776**

**The file contains technical information submitted after the application was filed and not included in this specification**

(73) Proprietor: **Takeda Chemical Industries, Ltd.**
**27, Doshomachi 2-chome Higashi-ku**
**Osaka-shi Osaka, 541 (JP)**

(72) Inventor: **Tetsuya, Okutani**
**3-5, Matsugaoka 4-chome**
**Takatsuki Osaka 569 (JP)**
Inventor: **Giichi, Goto**
**12-308, 1319 Makitacho**
**Takatsuki Osaka 569 (JP)**
Inventor: **Isuke, Imada**
**18-10, Tsuruyamadai 4-chome**
**Izumi Osaka 594 (JP)**
Inventor: **Masazumi, Watanabe**
**4-54, Seiwadainishi 2-chome**
**Kawanishi Hyogo 666-01 (JP)**

(74) Representative: **Lewin, John Harvey et al**
**Elkington and Fife High Holborn House 52/54**
**High Holborn London WC1V 6SH/GB**

**EP 0 021 841 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to new quinone compounds of value as medicines and to a novel method for producing quinone compounds.

Ubiquinones are effective against heart failure, periodontal disease, diabetes, muscular dystrophy, asthma, etc. but because they are generally low in water solubility, the route of administration is limited and the onset of action is not satisfactory. The research undertaken by us has resulted in the finding of new quinone compounds which possess desirable pharmacological activities and a novel method for producing a quinone compound, which is advantageous from the industrial point of view.

FR—A—2 269 333 and FR—A—2 293 194 disclose compounds of the formulae

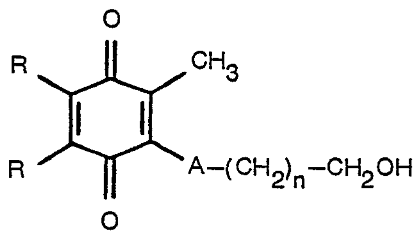

and

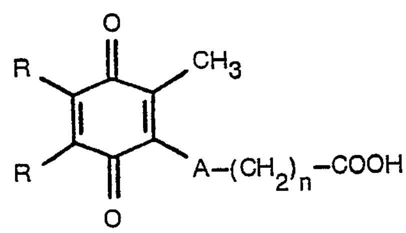

wherein R represents lower alkyl or lower alkoxy, A represents $-CH_2-$, $-CO-$ or $-\underset{OH}{\underset{|}{CH}}-$, *n* represents an integer of 1 to 8 and their esters. These compounds have pharmaceutical activities such as physiological host defence control activity, especially immuno-potentiating activity, and an action on the lysosomal membranes.

FR—A—2268778 and FR—A—2272068 disclose processes for the preparation of p-quinones by the reaction of oxygen with a phenol in the presence of a cobalt (II) coordination compound. US—A—3966776 relates to a process for preparing a parabenzoquinone in which an alkyl phenol is oxidised with oxygen or an oxygen-containing gas in the presence of a cobalt complex. US—A—3859317 describes a process for the preparation of parabenzoquinone by reacting phenol with oxygen in the presence of a cobalt or manganese complex.

One aspect of the present invention relates to a compound of the formula

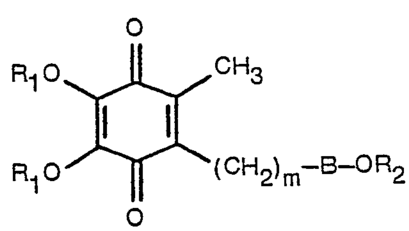

wherein *m* is an integer of 10 to 21; $R_1$ is lower alkyl having 1 to 4 carbon atoms; B is a $-CH_2-$ or $-CO-$ group; when B is $-CH_2-$, $R_2$ is hydrogen, lower alkyl having 1 to 4 carbon atoms or lower acyl having 1 to 8 carbon atoms, and when B is $-CO-$, $R_2$ is hydrogen or lower alkyl having 1 to 4 carbon atoms; provided that, where $R_1$ is methyl, B is $-CH_2-$ and $R_2$ is hydrogen, then *m* is 11 or 19 only, and excluding the following compounds:
2,3-dimethoxy-6-(12-methoxycarbonyldodecyl)-5-methyl-1,4-benzoquinone, and
6-(11-acetoxyundecyl)-2,3-dimethoxy-5-methyl-1,4-benzoquinone.

In the above formula (I), the lower alkyl designated by $R_1$ or $R_2$ is one having 1 to 4 carbon atoms, such as methyl, ethyl, n-propyl, n-butyl, etc. The lower acyl designated by $R_2$ is one having 1 to 8

carbon atoms. As embodiments of the lower acyl $R_2$, there are mentioned aliphatic acyl (e.g. formyl, acetyl, propionyl, butyryl, etc.) aromatoaliphatic acyl (e.g. phenylacetyl, etc.) and aromatic acyl (e.g. benzoyl, etc.). The integer *m* is preferably 10 to 17.

The present compound of the general formula (I) has the physiological activity of ubiquinones and, when the mitochondrial electron-transport activity of (I) was assayed by the general method for assaying the physiological activity of ubiquinones, it was found to display a marked degree of activity as shown in Table 1.

TABLE 1

Electron-transport activity of the present compound[1)]

| Compound | Level of addition (n moles) | Succinate oxidase activity [oxygen consumption per mg enzyme protein during 1 minutes (n atoms)] |
|---|---|---|
| Control | 0 | 19.34 ± 1.42 |
| [In formula I] m: 12 $R_1$:$CH_3$ $R_2$: H B:CO | 10 | 96.05 |
| [In formula I] m: 12 $R_1$:$CH_3$ $R_2$: $CH_3$ B:CO | 10 | 146.10 |
| [In formula I] m: 12 $R_1$:$CH_3$ $R_2$: H B:CO | 10 | 112.05 |

[1)] Ubiquinone-depleted enzyme preparations were prepared in accordance with Lester and Fleischer (Biochem. Biophys. Acta., *47*, 358—377, 1961). The electron-transport activity was determined based on succinate oxidase activity.

Furthermore, we investigated the effect of the present compound (I) on the production and release of the slow reacting substance of anaphylaxis (hereinafter, SRS—A) which has been mentioned as a main factor in asthma. Thus, in accordance with the method of Orange and Moore (J. Immunol., *116*, 392—397, 1976), the present compound (I) was added to guinea-pig lung fragments sensitized with egg albumin as the antigen, and the amount of SRS—A produced and released as a consequence was assayed by the method of Brocklehurst (J. Physiol., *151*, 416—435, 1960). It was found that, as shown in Table 2, the present compound displayed a very potent inhibitory action on SRS—A production at low concentration.

TABLE 2

The inhibitory effect of the present compound on SRS—A production

| Compound[1)] | Concentration ($\mu$M) | Inhibitory effect on SRS—A production[2)] (%) |
|---|---|---|
| [In formula I] | | |
| $R_1$: $CH_3$, B: CO $R_2$: H, m: 12 | 1 | 60.0 ± 3.3 |
| | 10 | 87.2 ± 5.5 |
| $R_1$: $CH_3$, B: CO $R_2$: H, m: 17 | 10 | 82.8 ± 4.4 |
| $R_1$: $CH_3$, B: CO $R_2$: H, m: 21 | 10 | 80.5 ± 0.5 |

[1)] The compound was added as dissolved in ethanol or dimethyl sulphoxide.

[2)] The inhibitory effect on SRS—A production was expressed as the % inhibition of production and release of SRS—A.

The present compound (I) has hypotensive, tissue metabolism-stimulating, immuno-regulatory, lysosomal membrane-stabilizing and SRA—A production inhibitory activities and is used as a heart failure remedy, cerebral circulation disturbance remedy, immuno-regulator, anti-allergic drug (for the treatment of e.g. asthma, rhinitis, etc.), etc. for mammalian animals including human beings. Useful dosage forms include capsules, granules, powders, tablets, troches, pills, syrups, injections, suppositories, etc. As an antiallergic drugs, dosage forms such as ointment, aerosol, inhalation mist, etc. may be employed.

The pharmaceutically acceptable materials which can be used in formulating pharmaceutical preparations include, among others, excipients such as sucrose, lactose, glucose, starch, mannitol, sorbitol, cellulose, talc, cyclodextrin, etc.; binders such as cellulose, methylcellulose, polyvinyl pyrrolidone, gelatin, gum arabic, polyethylene glycol, sucrose, starch, etc.; disintegrators such as starch, carboxymethylcellulose, carboxymethylcellulose calcium salt, etc.; lubricants such as talc; flavourants; preservatives such as sodium benzoate; suspending agents such as methylcellulose, polyvinyl pyrrolidone, aluminium stearate, polysorbate 80, Emulgen 408, Emasol 310, etc., (Emulgen 408 and Emasol 310 are Trade Marks) solvents such as water, and pharmaceutical bases such as cacao butter, polyethylene glycol, witepsol, white petroleum, etc. These materials are selectively used according to the type of pharmaceutical preparation.

The compound (I) of this invention is admininstered orally or otherwise, preferably at doses of 5 to 500 mg daily (0.1 to 10 mg/kg) for adult humans, for instance.

The other aspect of the present invention relates to a method for producing a compound of the formula:

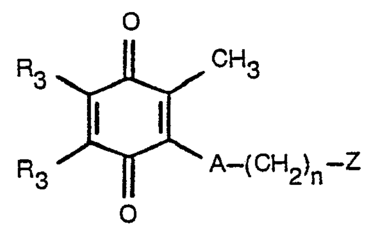
(II)

[wherein each $R_3$ is lower alkyl having 1 to 4 carbon atoms or lower alkoxy having 1 to 4 carbon atoms, or two $R_3$'s taken together represent $—CH=CH—CH=CH—$; *n* is 0 or an integer of 8 to 20 and then *n* is 8 to 20, A is $—CH_2—$,

$$\begin{array}{c}—CH— \\ | \\ OH\end{array}$$

or —CO— group and Z is $CH_2CH$, acyloxy methyl having 2 to 11 carbon atoms esterified- or unesterified carboxyl having 1 to 5 carbon atoms, lower alkoxy methyl having 2 to 5 carbon atoms, silyloxymethyl having 4 to 7 carbon atoms or acetalmethyl having 3 to 8 carbon atoms; when *n* is 0, $—A—(CH_2)_n—Z$ is

$$\begin{array}{c}—(CH_2—CH=\underset{\displaystyle CH_3}{\underset{|}{C}}—CH_2)_l—Z\end{array}$$

(wherein *l* is an integer of 3 to 12) and Z is hydrogen], which comprises oxidizing a compound of the formula:

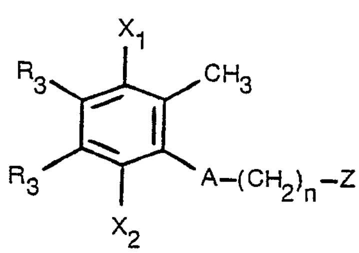
(III)

(wherein either $X_1$ or $X_2$ is hydroxyl or amino, with the other being hydrogen or an esterified hydroxyl group having 1 to 8 carbon atoms or etherified hydroxyl group having 2 to 8 carbon atoms; the other symbols are as defined above) with oxygen or air in the presence of a cobalt-complex of the formula:

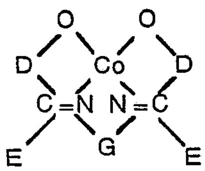

(IV)

[wherein D is a vinylene group of

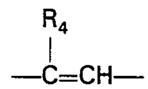

($R_4$ is lower alkyl or phenyl) or an orthophenylene group of

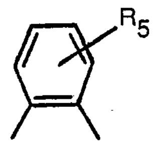

($R_5$ is H, hydroxy or lower alkoxy); E is H or lower alkyl; G is an ethylene group of

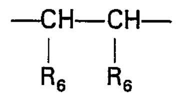

($R_6$ is H or lower alkyl) or an orthophenylene group of

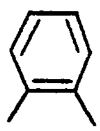

].

It is known that a quinone derivative of general formula (II) can be obtained by oxidizing a compound of general formula (III) with an oxidizing agent such as hydrogen peroxide, peracetic acid, performic acid, potassium dichromate, potassium permanganate, chromic anhydride, potassium nitrosodisulphate, potassium persulphate and so on. However, when an oxidizing agent such as those mentioned above is employed, the desired compounds can be obtained only in low yield and the byproducts are produced in large amounts so that the desired compounds are hardly of high purity. Moreover, using the above-mentioned oxidizing agents in large amounts is not desirable from the safety point of view. Thus, the conventional processes are not suited for commercial production purposes. In contrast, the method of this invention employs a complex compound of cobalt and yields the desired compounds in good yield and purity, thus being very advantageous for commercial-scale production.

The method according to the invention excludes the production of 6-(22-hydroxydocosyl)-2,3-dimethoxy-5-methyl-1,4-benzoquinone by oxidation of 6-(22-hydroxydocosyl)-2,3-dimethoxy-5-methyl phenol in the presence of bis(4-hydroxysalicylidine)ethylenediimino-cobalt (II).

Referring to the above general formulae (II) and (III), the lower alkyl group $R_3$ may be a straight-chain or branched alkyl group containing 1 to 4 carbon atoms each, such as methyl, ethyl, n-propyl, i-propyl, n-butyl, i-butyl, etc. The alkoxy group, also designated by $R_3$, may be a group of 1 to 4 carbon atoms, such as methoxy, ethoxy, n-propoxy, n-butoxy, etc. The symbol *n* is 0 or an integer of 8 to 20, preferably, 0 or 10 to 20, and most preferably 13 to 20. The symbol *l* is an integer of 3 to 12. The lower acyloxymethyl group Z is one having 2 to 11 carbon atoms, and may be an alkylcarbonyloxymethyl group of 3 to 6 carbon atoms, preferably of 3 to 5 carbon atoms, e.g. acetyloxymethyl, n-propionyloxymethyl, n-butyryloxymethyl etc.; an arylcarbonyloxymethyl group of 8 or 9 carbon atoms, e.g. phenylcarbonyloxymethyl, p-methylphenylcarbonyloxymethyl, etc.; or an aralkylcarbonyloxymethyl group of 9 to 11 carbon atoms, e.g. benzylcarbonyloxymethyl, phenethylcarbonyloxymethyl etc. The optionally esterified carboxyl group Z may, for example, be a free carboxyl group or an alkoxycarbonyl group of 2 to 5 carbon atoms, e.g. methoxycarbonyl, ethoxycarbonyl, n-propoxycarbonyl, n-butoxycarbonyl, etc. The lower alkoxymethyl group Z may be a group of 2 to 5 carbon atoms, e.g. methoxymethyl, ethoxymethyl, n-propoxymethyl, n-butoxymethyl, etc. The silyloxy methyl group Z may be a group having 4 to 7 carbon atoms, e.g. trimethylsilyloxymethyl, t-butyldimethylsilyloxymethyl, etc. The acetalmethyl group Z may be a group having 3 to 8 carbon atoms e.g. tetrahydropyranyloxymethyl, tetrahydrofuranyloxymethyl, methoxymethyloxymethyl, methylthiomethyloxymethyl, etc. The esterified or etherified hydroxyl group, which is designated by $X_1$ or $X_2$, may for example be an acyloxy group, such as alkylcarbonyloxy groups of 2 to 6 carbon atoms, preferably of 2 to 4 carbon atoms (e.g. acetyloxy, propionyloxy, butyryloxy, etc.), arylcarbonyloxy groups having 6 to 8 carbon atoms (e.g. benzoyl-

oxy), etc., tetrahydropyranyloxy, tetrahydrofuranyloxy, alkoxyalkyloxy groups having 2 to 8 carbon atoms such as methoxymethyloxy, 2-methoxymethyloxy, etc., silyloxy groups having 3 to 6 carbon atoms such as trimethylsilyloxy, t-butyldimethylsilyloxy, etc.

The oxidation reaction according to this invention is carried out by catalytic oxidation with oxygen, air or the like in the presence of a cobalt complex compound of formula (IV). The cobalt complex compound is able to yield an oxygen complex compound reversibly on interaction with oxygen. In formula (IV), the lower alkyl groups $R_4$, $R_6$ and E are ones having 1 or 2 carbon atoms, e.g. methyl, ethyl, etc.; the lower alkoxy group $R_5$ is one having 1 or 2 carbon atoms and may for example be methoxy or ethoxy. As examples of compounds having the above general formula (IV), there may be mentioned divalent cobalt-Schiff's base complexes such as Cobalt salen (salcomine): bis(salicylidene)ethylenediiminocobalt (II), Cobalt salphen: bis(salicylidene)-o-phenylenediiminocobalt (II), Cobalt acacen: bis-(acetonylacetylidene)ethylenediiminocobalt (II), Cobalt bzacen: bis(phenacylacetylidene)ethylenediiminocobalt (II), and other compounds such as bis(3-alkoxysalicylidene)ethylenediiminocobalt (II), bis(4-hydroxysalicylidene)ethylenediiminocobalt (II), etc.

This catalytic oxidation reaction is usually conducted in an organic solvent. The solvent includes, for example, halogenated hydrocarbons such as dichloromethane, 1,2-dichloroethane, 1,1,2,2-tetrachloroethane, chloroform, carbon tetrachloride, etc.; lower alcohol-type organic solvents such as methanol, ethanol, butanol, etc.; amide-type solvents such as dimethylformamide, dimethylacetamide, hexamethylphosphorotriamide, etc.; pyridine and substituted pyridine (e.g. s-collidine), dimethylsulphoxide, sulpholane, etc. These organic solvents can be used as a mixture of two or more species.

The amount of metal complex compound used in accordance with this invention should vary with the type of substrate compound and other conditions of reaction. Usually, the complex compound is used in a range of from 0.01 to 50 weight percent relative to compound (III) and, preferably, 1 to 30 weight percent on the same basis. The oxidation reaction with the aid of this complex compound is promoted by the addition of imidazole, pyridine or cyanide, ions (e.g. sodium cyanide). The amount of such an additive is preferably the same as that of the transition metal complex.

The reaction temperature is normally in the range of from 0°C to 80°C and, preferably, room temperature. This reaction may be conducted at atmospheric pressure and, optionally, at an elevated pressure not over $1.52 \times 10^7$ $Nm^{-2}$ (150 atm). The reaction time may range from 0.5 hours to 30 days, normally up to 75 hours.

After the oxidation reaction, the product compound can be easily isolated and purified by procedures known *per se* (e.g. extraction, concentration, recrystallization, chromatography, etc.).

The compound (I) can be prepared by oxidizing the compound (III) wherein $R_3$ is alkoxy; A is $-CH_2-$; Z is hydroxymethyl, alkoxymethyl, acyloxymethyl, carboxyl or alkoxycarbonyl, and *n* is an integer of 9 to 20.

The starting compound (III) used in the practice of the present invention can be produced, for example, by the method described in US Patent No. 4,139,545 or a method analogous thereto. The compound (III) can also be produced through the following steps.

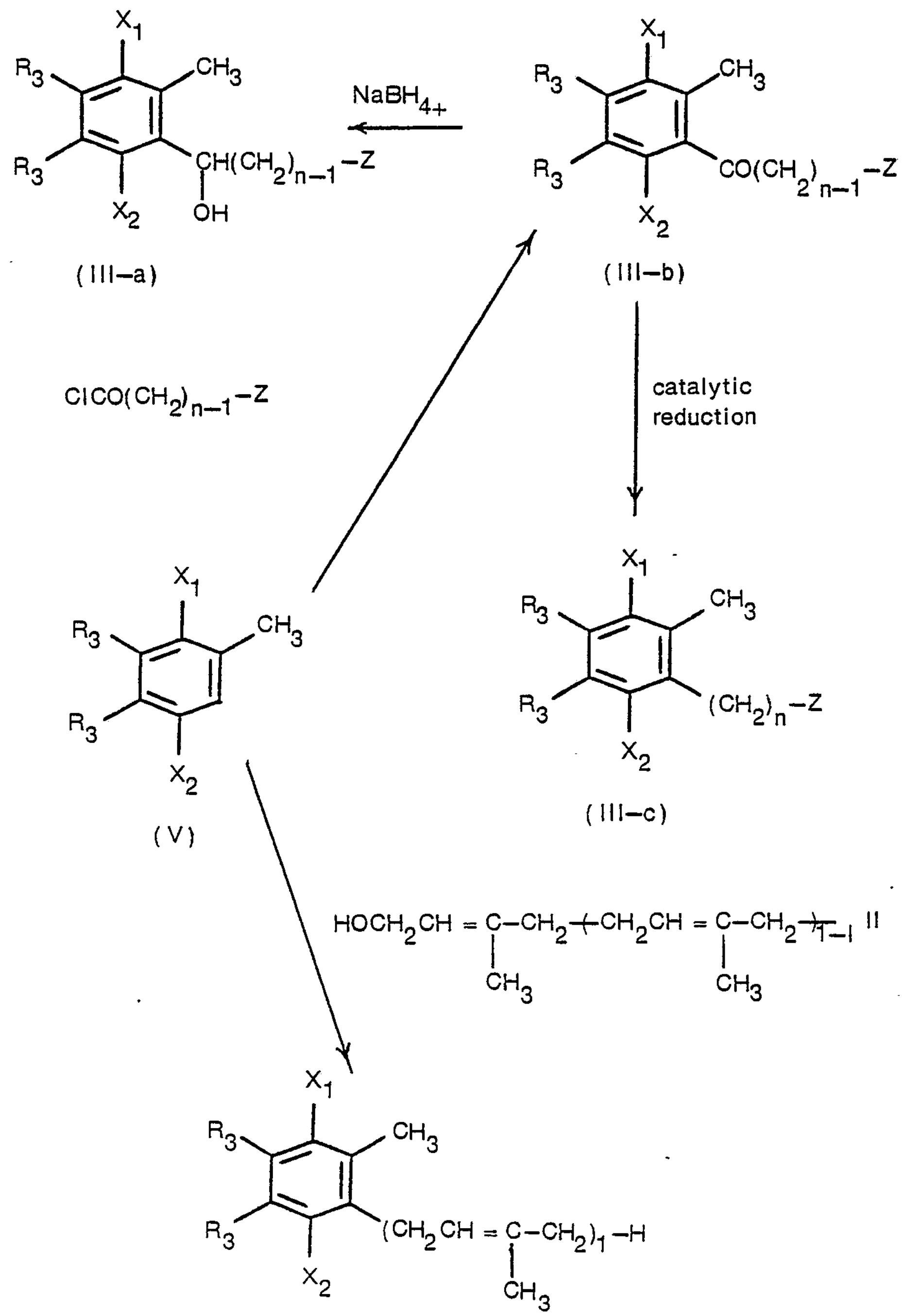


(III-a) (III-b) (V) (III-c) II (III-d)

In the above formulae, each symbol is as defined hereinbefore.

The invention is illustrated by the following Examples.

Example 1

11.7 g of methyl 13-chloroformyltridecanoate are dissolved in 200 ml of petroleum ether and, under ice-cooling and stirring, 60 ml of ice-water are added, followed by addition of 5.2 of sodium peroxide in small portions. The mixture is stirred for an hour, at the end of which time it is extracted with ether. The extract is washed with water and dried over calcium chloride, and the solvent is distilled off under reduced pressure. The above procedure yields 7.6 g of crude crystals of bis-13-methoxy-carbonyltridecanoyl peroxide. This product is subjected to the next reaction without being purified. IR $\nu_{max}^{film}$ $cm^{-1}$: 1790,

$$1760\,(-\overset{\overset{O}{\|}}{C}-O-O),$$

1730 ($COOCH_3$). 1.7 g of 2,3-dimethoxy-5-methyl-1,4-benzoquinone are dissolved in 20 ml of acetic acid and, under stirring at 90°C, 7.6 g of bis-13-methoxycarbonyltridecanoyl peroxide are added in small portions. The mixture is heated for 22 hours and, after cooling, is diluted with water and extracted with ether. The extract is washed with a saturated aqueous solution of sodium chloride, aqueous sodium hydrogen carbonate and aqueous sodium chloride in that order, followed by drying. The solvent is distilled off under reduced pressure and the residue is recrystallized from hexane to obtain 1.37 g of 2,3-dimethoxy-6-(12-methoxycarbonyldodecyl)-5-methyl-1,4-benzoquinone. m.p. 54°C.

Elemental analysis for $C_{23}H_{36}O_6$

| | | |
|---|---|---|
| Calcd. | C, 67.62; | H, 8.88 |
| Found | C, 67.52 | H, 8.59 |

## Example 2

1.5 g of 2,3-dimethoxy-6-(12-methoxycarbonyldodecyl)-5-methyl-1,4-benzoquinone are dissolved in 200 ml of ether, and the solution is shaken with 75 ml of a 20% aqueous solution of sodium hydrosulphite. The ether layer is separated and concentrated. To the residue are added, under ice-cooling and in a nitrogen gas stream, 5 ml of a 30% aqueous solution of sodium hydrosulphite and 3.5 ml of a 30% aqueous solution of potassium hydroxide. The mixture is warmed at 50°C for 2.5 hours. After cooling, the reaction mixture is made acidic with cold hydrochloric acid and extracted with ether. The ether is distilled off under reduced pressure, the residue is dissolved in 75 ml of methanol and a solution of 15 g of ferric chloride in 60 ml of water is added. The mixture is stirred at room temperature for 1 hour, at the end of which time it is diluted with water and extracted with ethyl acetate. The extract is washed with water and dried, and the solvent is distilled off under reduced pressure. The residue is crystallized from ether-hexane to obtain 1.09 g of 6-(12-carboxydodecyl)-2,3-dimethoxy-5-methyl-1,4-benzoquinone. m.p. 62°C.

Elemental analysis for $C_{22}H_{34}O_6$

| | | |
|---|---|---|
| Calcd. | C, 66.98; | H, 8.69 |
| Found | C, 67.15; | H, 8.77 |

## Example 3

(a) 1 g of monomethyl-1,20-eicosanedicarboxylate is dissolved in 5 ml of thionyl chloride, and after stirring at room temperature for 12 hours, the excess thionyl chloride is distilled off to recover crude crystals of methyl 21-chloroformylheneicosanoate. This product is subjected to the next reaction without being purified. IR $\nu_{max}^{KBr}$ $cm^{-1}$: 1790 (COCl), 1730 ($COOCH_3$).

8 g of methyl 21-chloroformylheneicosanoate are dissolved in 60 ml of 1,2-dichloroethane followed by addition of 5.5 g of aluminium chloride. The mixture is stirred. Then, under ice-cooling and stirring in a nitrogen stream, a solution of 5.67 g of 3,4,5-trimethoxytoluene in 1,2-dichloroethane (13 ml) is added dropwise. The mixture is stirred for 48 hours, after which 1.3 g of aluminium chloride is added. The mixture is stirred at room temperature for 16 hours and further at 35°C—38°C for 40 minutes. To this reaction mixture are added ice-water and 3N—HCl, followed by extraction with chloroform. The extract is washed with water, a saturated aqueous solution of sodium hydrogen carbonate and water in that order. After drying, the solvent is distilled off under reduced pressure and the residue is chromatographed on silica gel. The fractions containing the desired compound are pooled and recrystallized from methanol. The above procedure yields methyl 21-(2-hydroxy-3,4-dimethoxy-6-methylbenzoyl)heneicosanoate as colourless needles. m.p. 73—74°C. NMR ($\delta$, in deuteriochloroform): 1.24 (36H, b, $CH_2$), 2.27 (2H, t, $CH_2COO$), 2.42 (3H, s, nuclear $CH_3$), 2.86 (2H, t, $COCH_2$), 3.64 (3H, s, $COOCH_3$), 3.84 (3H, s, $OCH_3$), 3.87 (3H, s, $OCH_3$), 6.22 (1H, s, nuclear H).

Perchloric acid is added to a solution of methyl 21-(2-hydroxy-3,4-dimethoxy-6-methylbenzoyl)-heneicosanoate in acetic acid, and catalytic reduction is carried out with 5% palladium-On-carbon. The reaction mixture is worked up as in the corresponding step of Example 5 and the resultant product is recrystallized from ethanol. The above procedure yields methyl 22-(2-hydroxy-3,4-dimethoxy-6-methylphenyl)-docosanate as colourless needles. m.p. 71°C—72.5°C.

300 mg of methyl 22-(2-hydroxy-3,4-dimethoxy-6-methylphenyl)docosanoate followed by addition of 20 ml of a 14% aqueous solution of sodium hydroxide. The mixture is stirred at 50°C—60°C for 1.5 hours. The reaction mixture is made acidic with 3N—HCl and extracted with ethyl acetate. The extract is washed with water and, after drying, the solvent is distilled off under reduced pressure. The residue is recrystallized from methanol to obtain 22-(2-hydroxy-3,4-dimethoxy-6-methylphenyl)docosanoic acid. m.p. 67°C—68°C.

b) In the presence of triethylamine (14.3 ml), 6-(9-chloroformylnonyl)-2,3-dimethoxy-5-methylphenyl acetate (36.9 g) is reacted with 1-morpholino-1-cyclododecene (24.3 g) as in the corresponding step of Example 6. The reaction product is treated with 6N—HCl (47 ml) and the resultant 2-[10-(2-acetoxy-3,4-dimethoxy-6-methylphenyl)-decanoyl]cyclododecanone (46.3 g) is hydrolyzed with a 6N-aqueous solution of sodium hydroxide to obtain 32.4 g of 13-oxo-22-(2-hydroxy-3,4-dimethoxy-6-methylphenyl)docosanoic acid, m.p. 63°C—64°C.

13-Oxo-22-(2-hydroxy-3,4-dimethoxy-6-methylphenyl)-docosanoic acid (5.21 g) is reduced in the presence of p-toluenesulphonic acid (250 mg) and p-toluenesulphonyl hydrazide (2.33 g) in a solvent mixture of dimethylformamide (25 ml)-sulpholane (25 ml)-cyclohexane (25 ml) as in the corresponding step of Reference Example 6. The resultant crude product is recrystallized from methanol to obtain 2.97 g of 22-(2-hydroxy-3,4-dimethoxy-6-methylphenyl)docosanoic acid, m.p. 67°C—68°C. The physico-chemical constants of this product are all in agreement with those of the crystals obtained in the above step a).

To a solution of 300 mg of 22-(2-hydroxy-3,4-dimethoxy-6-methylphenyl)docosanoic acid in 20 ml of N,N-dimethylformamide are added 50 mg of bis(salicylidene)ethylenediiminocobalt (II) and the mixture is stirred in an oxygen gas stream at room temperature for 24 hours. The insolubles are filtered off and the filtrate is concentrated under reduced pressure. The residue is dissolved in ethyl acetate, washed with water and dried. The solvent is distilled off and the residue is dissolved in chloroform and column-chromatographed on silica gel, elution being carried out with chloroform. The eluate is recrystallized from ethanol to obtain 6-(21-carboxyheneicosyl)-2,3-dimethoxy-5-methyl-1,4-benzoquinone as orange-yellow needles melting at 86°C—87°C.

Elemental analysis for $C_{31}H_{52}O_6$

| | | |
|---|---|---|
| Calcd. | C, 71.50; | H, 10.07 |
| Found | C, 71.56; | H, 10.06 |

Example 4

Aluminium chloride (7 g) is added to a solution of 18-acetoxy-n-octadecanoyl chloride (11 g) in 1,2-dichloroethane (50 ml), and the mixture is stirred at room temperature for 2 hours. This reaction mixture is cooled to 5°C and a solution of 3,4,5-trimethoxytoluene (6.2 g) in 1,2-dichloroethane (20 ml) is added. The mixture is stirred at room temperature for 72 hours. Then, this reaction mixture is heated to 50°C—60°C and stirred for 30 minutes. After cooling, ice-water is added to the reaction mixture and the product is extracted with dichloromethane. The dichloromethane layer is washed with water and the solvent is distilled off to recover an oil (12.1 g). This oil is dissolved in methanol (150 ml) followed by addition of sodium hydroxide (5.2 g). The mixture is stirred at room temperature for 2 hours, at the end of which time it is neutralized with 5N—HCl and the solvent is distilled off. The resultant crude crystals are rinsed with water and recrystallized from dichloromethane-ether (1:1). The above procedure yields 6-(18-hydroxy-1-oxooctadecyl)-2,3-dimethoxy-5-methylphenol (6.4 g) as colourless needles, m.p. 101°C.

5% palladium-on-carbon (50% hydrous) (0.5 g) and 70% perchloric acid (0.05 ml) are added to a solution of 6-(18-hydroxy-1-oxooctadecyl)-2,3-dimethoxy-5-methylphenol (1.4 g) in acetic acid (30 ml) and catalytic reduction is carried out at atmospheric temperature and pressure. After the absorption of hydrogen has subsided, the catalyst is filtered off and the filtrate is concentrated under reduced pressure, whereupon a colourless oil is obtained. This product is dissolved in either and the ether layer is washed with 5% aqueous solution of sodium hydrogen carbonate and dried over anhydrous sodium sulphate. The solvent is then distilled off and the resultant crude crystals are recrystallized from hexane. The above procedure yields 6-(18-acetoxyoctadecyl)-2,3-dimethoxy-5-methylphenol (1.4 g) as colourless needles, m.p. 53°C.

The above 18-acetoxy compound was deacetylated by the corresponding procedure of Example 5 and the resultant 6-(18-hydroxyoctadecyl)-2,3-dimethoxy-5-methylphenol (0.5 g) is dissolved in dimethylformamide (1 l). To this solution is added potassium nitrosodisulphonate (13 g), 700 ml of water, 100 ml of methanol and monopotassium phosphate (1 g), and the mixture is stirred at room temperature for 45 days. The product is extracted in the conventional manner and recrystallized from ether-hexane. The above procedure yields 6-(18-hydroxyoctadecyl)-2,3-dimethoxy-5-methyl-1,4-benzoquinone as yellow needles (0.31 g), m.p. 81°C.

Elemental analysis for $C_{27}H_{46}O_5$

| | | |
|---|---|---|
| Calcd. | C, 71.96; | H, 10.29 |
| Found | C, 72.06; | H, 10.27 |

172 mg of 6-(18-hydroxyoctadecyl)-2,3-dimethoxy-5-methyl-1,4-benzoquinone are dissolved in 10 ml of acetone. Separately, 2.672 g of chromic acid anhydride are dissolved in 2.3 ml of concentrated sulphuric acid and diluted with water to 10 ml. This diluted solution (0.5 ml) is added to

the above acetone solution and the mixture is stirred under ice-cooling for 10 minutes. The reaction mixture is diluted with water and extracted with chloroform. The extract is washed with water and dried, and the solvent is distilled off under reduced pressure. The residue is column-chromatographed on silica gel, elution is carried out with chloroform and the eluate is recrystallized from ethanol. The above procedure yields 65 mg of 6-(17-carboxyheptadecyl)-2,3-dimethoxy-5-methyl-1,4-benzoquinone as orange-yellow crystals, m.p. 77—79°C.

Elemental analysis for $C_{27}H_{44}O_6$

| | | |
|---|---|---|
| Calcd. | C, 69.79; | H, 9.55 |
| Found | C, 69.86; | H, 9.78 |

Example 5

Aluminium chloride (28 g) is added to a solution of 11-acetoxy-n-undecanoyl chloride (27.6 g) in 1,2-dichloroethane (150 ml), and the mixture is stirred at room temperature for 2 hours. The reaction mixture is cooled to 5°C and, then, a solution of 3,4,5-trimethoxytoluene (19.1 g) in 1,2-dichloroethane (50 ml) is added. The mixture is stirred at room temperature for 72 hours. The reaction mixture is heated to 50°C—60°C and further stirred for 30 minutes. After cooling, 300 ml of ice-water are added to the reaction mixture and the product is extracted with dichloromethane. The dichloromethane layer is washed with water and dried over anhydrous magnesium sulphate. The solvent is then distilled off to recover 6-(11-acetoxy-1-oxoundecyl)-2,3-dimethoxy-5-methylphenol (35 g). IR $\nu_{max}^{Neat}$ $cm^{-1}$: 1730 (OAc), 1680 (CO), 1610, 1580 (Ar). MS m/e: 394 ($M^+$), 352, 334, 195.

Sodium hydroxide (7 g) is added to a solution of 6-(11-acetoxy-1-oxoundecyl)-2,3-dimethoxy-5-methylphenol (34 g) in methanol (300 ml) and the mixture is stirred at room temperature for 2 hours. The reaction mixture is neutralized with 5N—HCl and the solvent is distilled off, thereby leaving crude crystals. These crystals are rinsed with water and recrystallized from ether-hexane (1:1). The above procedure yields colourless needles of 6-(11-hydroxy-1-oxoundecyl)-2,3-dimethoxy-5-methylphenol (30 g), m.p. 81°C.

5% palladium-on-carbon (50% hydrous) (3 g) and 70% perchloric acid (0.1 ml) are added to a solution of 6-(11-hydroxy-1-oxoundecyl)-2,3-dimethoxy-5-methylphenol (14 g) in acetic acid (200 ml), and catalytic reduction is carried out at atmospheric temperature and pressure. When the hydrogen has ceased to be absorbed, the catalyst is filtered off and the filtrate is concentrated under reduced pressure. The residue is extracted with dichloromethane and the dichloromethane layer is washed with a 5% aqueous solution of sodium hydrogen carbonate and dried over anhydrous magnesium sulphate. The solvent is then distilled off to obtain a colourless oil of 6-(11-acetoxyundecyl)-2,3-dimethoxy-5-methylphenol (15 g). IR $\nu_{max}^{Neat}$ $cm^{-1}$: 3450 (OH), 1730 (OAc), 1610, 1580 (Ar). NMR $\delta_{ppm}^{CDCl_3}$: 1.1—1.9 [18H, m, $—(CH_2)_9—$], 2.02 (3H, s,) Ac), 2.22 (3H, s, $C_5—CH_3$), 2.54 (2H, t, J=7Hz, $C_1—H_2$), 3.79 (3H, s, $OCH_3$), 3.83 (3H, s, $OCH_3$), 4.01 (2H, t, J=6Hz, $—CH_2$ OAc), 5.78 (1H, s, $C_1—OH$), 6.23 (1H, s, $C_4—H$). MS m/e: 380 ($M^+$), 338, 181.

Potassium nitrosodisulphonate (24 g), water (400 ml), methanol (30 ml) and monopotassium phosphate (1.0 g) are added to a solution of 6-(11-acetozyundecyl)-2,3-dimethoxy-5-methylphenol (8 g) in N,N-dimethylformamide (400 ml). The mixture is stirred at room temperature for 28 days. The product is extracted with dichloromethane and the dichloromethane layer is washed with water and dried over anhydrous magnesium sulphate. The solvent is then distilled off and the resultant crude crystals are recrystallized from hexane. The above procedure yields 6-(11-acetoxyundecyl)-2,3-dimethoxy-5-methyl-1,4-benzoquinone (6.4 g) as orange-yellow needles, m.p. 41°C.

To a solution of 6-(11-acetoxyundecyl)-2,3-dimethoxy-5-methyl-1,4-benzoquinone (4.2 g) in methanol (200 ml) is added concentrated hydrochloric acid (0.1 ml) and the mixture is allowed to stand at room temperature for 12 hours.

Sodium hydrogen carbonate (0.2 g) is added to the reaction mixture and the solvent is distilled off. The product is dissolved in dichloromethane, the insolubles are filtered off and the dichloromethane is distilled off. The resultant crude crystals are recrystallized from hexane ether (3:1) to obtain 6-(11-hydroxyundecyl)-2,3-dimethoxy-5-methyl-1,4-benzoquinone as orange-coloured needles (3.6 g), m.p. 57°C.

6-(11-Hydroxyundecyl)-2,3-dimethoxy-5-methyl-1,4-benzoquinone is reacted in the same manner as in Example 4 and the product is purified by silica gel column chromatography to recover orange-yellow needles of 6-(10-carboxydecyl)-2,3-dimethoxy-5-methyl-1,4-benzoquinone. m.p. 50°C—52°C. NMR ($\delta$, in deuteriochloroform): 1.32 (16H, b, $CH_2$), 2.03 (3H, s, $CH_3$), 2.18—2.65 (4H, m, $CH_2COO$, $CH_2$ on the ring), 4.02 (6H, s, $OCH_3$), 5.35 (1H, b, COOH).

Example 6

10-(2-hydroxy-3,4-dimethoxy-6-methylphenyl)-decanoic acid (33.8 g) is suspended in acetic anhydride (100 ml) followed by addition of concentrated sulphuric acid (5 ml). The mixture is reacted at room temperature for 1.5 hours, at the end of which time the solvent is distilled off. To the residue are

added dioxane (250 ml) and a 5% aqueous solution of sodium hydrogen carbonate (250 ml), and the mixture is stirred at room temperature for 24 hours. The reaction mixture is adjusted to pH 3 with 7N—HCl and extracted by the addition of ethyl acetate (300 ml) and water (500 ml). The ethyl acetate layer is washed with water and dried over anhydrous sodium sulphate. The solvent is distilled off to recover 36.2 g of 10-(2-acetoxy-3,4-dimethoxy-6-methylphenyl)decanoic acid as an oil. This oil is dissolved in anhydrous benzene (50 ml) and under ice-cooling, oxalyl chloride (25 ml) is added dropwise. Following the dropwise addition and after bubbles have subsided, the reaction is allowed to proceed at 50°C for an hour. The solvent is then distilled off, toluene is added and the solvent is distilled off under reduced pressure. The residue is dried over sodium hydroxide in a desiccator to obtain 36.9 g of 6-(9-chloroformylnonyl)-2,3-dimethoxy-5-methylphenyl acetate. NMR ($\delta$, in deuteriochloroform): 1.33 (14H, b, $CH_2$), 2.33, 2.37 (6H, d, $COCH_3$, nuclear $CH_3$), 2.95 (4H, t, $CH_2CO$, nuclear $CH_2$), 3.87, 3.90 (6H, s, $OCH_3$) 6.73 (1H, s, nuclear H). IR $\nu_{max}^{film}$ $cm^{-1}$: 1800 (COCl), 1770 ($OCOCH_3$). 6-(9-chloroformylnonyl)-2,3-dimethoxy-5-methylphenyl acetate (5.0 g) is dissolved in chloroform (4 ml), and under ice-cooling, the solution is added dropwise to a mixture of 1-morpholino-1-cyclohexene (2.51 g) and triethylamine (1.93 ml). After the dropwise addition has been completed, the reaction is allowed to proceed at room temperature for 90 hours. To this reaction mixture is added 6N-hydrochloric acid (7.5 ml), and with vigorous stirring, the mixture is refluxed for 5 hours. The mixture is subjected to extraction by the addition of chloroform (20 ml) and water (20 ml). The water layer is further extracted with chloroform (20 ml) and the chloroform layers are combined, washed with water and dried over anhydrous sodium sulphate. The solvent is distilled off and the residue is dissolved again in chloroform and subjected to chromatography on silica gel (60 g), elution being carried out with chloroform. The fractions containing the desired compound are pooled and the solvent is distilled off to obtain 3.2 g of 2-[10-(2-acetoxy-3,4-dimethoxy-6-methylphenyl)decanoyl]cyclohexanone as an oil. This oil is dissolved in ethanol (3.5 ml) followed by addition of a hot 6N-aqueous solution of sodium hydroxide (3.5 ml). The mixture is heated under reflux for 10 minutes. Then, at room temperature, the reaction mixture is adjusted to pH 2 with 6N—HCl, followed by extraction by the addition of ethyl acetate (30 ml) and water (30 ml). The water layer is further extracted with ethyl acetate (10 ml) and the ethyl acetate layers are combined, washed with water and dried over anhydrous sodium sulphate. The solvent is distilled off and the residue is recrystallized from ether-petroleum ether. The above procedure yields, 1.96 g of 7-oxo-16-(2-hydroxy-3,4-dimethoxy-6-methylphenyl)hexadecanoic acid, m.p. 55—57°C.

To a solvent mixture of dimethylformamide (10 ml) and sulpholane (10 ml) containing p-toluenesulphonic acid (100 mg) is added 7-oxo-16-(2-hydroxy-3,4-dimethoxy-6-methylphenyl) hexadecanoic acid (1.75 g) and p-toluenesulphonyl hydrazide (930 mg) at 100°C. After 5 minutes, cyclohexane (10 ml) and sodium cyanoborohydride (1.0 g) are added. The reaction is conducted at 100°C for 20 minutes. Then, at room temperature, a saturated aqueous solution of sodium chloride (100 ml) is added, followed by extraction with ether (50 ml × 2). The ether layers are combined, washed with water, 1N-HCl and water in that order, and dried over anhydrous sodium sulphate. The solvent is distilled off, and the residue is dissolved in chloroform and subjected to column chromatography on silica gel, elution being carried out with chloroform. The fractions containing the desired compound are pooled and the solvent is distilled off. To the crystalline residue is added petroleum ether and, after cooling, the crystals are collected by filtration. The above procedure yields 970 mg of 16-(2-hydroxy-3,4-dimethoxy-6-methylphenyl)-hexadecanoic acid, m.p. 45°C—46°C.

16-(2-hydroxy-3,4-dimethoxy-6-methylphenyl)-hexadecanoic acid (844 mg) is dissolved in dimethylformamide (20 ml), followed by addition of bis(salicylidene)ethylenediiminocobalt (II) (163 mg). The mixture is stirred in a stream of oxygen gas at room temperature for 24 hours. The solvent is distilled off, the residue is dissolved in ethyl acetate (100 ml) and the insolubles are filtered off. The filtrate is washed with 1N—HCl and water and dried over anhydrous sodium sulphate. The solvent is distilled off and the residue is dissolved in a small amount of chloroform and purified by column chromatography on silica gel (20 g) with chloroform as the eluent. The fractions containing the product compound are pooled, the solvent is distilled off and the residue is recrystallized from ether-petroleum ether to obtain 514 mg of 6-(15-carboxypentadecyl)-2,3-dimethoxy-5-methyl-1,4-benzoquinone. m.p. 70°C—71°C.

Elemental analysis for $C_{25}H_{40}O_6$

| | | |
|---|---|---|
| Calcd. | C, 68.77; | H, 9.23 |
| Found | C, 68,89; | H, 9.30 |

## Example 7

Aluminium chloride (8.2 g) is added to a solution of 12-acetoxy-n-dodecanoyl chloride (8.5 g) in 1,2-dichloroethane (30 ml), and the mixture is stirred at room temperature for 2 hours. The mixture is cooled to 5°C and a solution of 3,4,5-trimethoxytoluene (5.6 g) in 1,2-dichloroethane (20 ml) is added to the above mixture. The whole mixture is stirred at room temperature for 72 hours, and at

50°C—60°C for 30 minutes. Methanol (200 ml) is added to the reaction mixture and the mixture is stirred at 50°C for 3 hours. The solvent is distilled off, and the residue is subjected to extraction with dichloromethane. The dichloromethane layer is washed with water and dried over magnesium sulphate. The solvent is distilled off, thereby leaving crude crystals. The product is recrystallized from ether-hexane (1:1) to give 6-(12-hydroxy-1-oxododecyl)-2,3-dimethoxy-5-methylphenol (8.5 g) as colourless needles. Melting point 82°C.

5% palladium carbon (50% hydrous) (1.1 g) and 70% perchloric acid (0.1 ml) are added to a solution of 6-(12-hydroxy-1-oxododecyl)-2,3-dimethoxy-5-methylphenol (6.4 g) in acetic acid (150 ml), and the mixture is subjected to catalytic reduction at atmospheric temperature and pressure. After the absorption of hydrogen has been completed, the catalyst is filtered off and the filtrate is concentrated under reduced pressure. The residue is extracted with dichloromethane and the dichloromethane layer is washed with a 5% aqueous solution of sodium hydrogen carbonate and dried over anhydrous magnesium sulphate. The solvent is distilled off to give 6-(12-acetoxydodecyl)-2,3-dimethoxy-5-methylphenol, (6.8 g) as a colourless oil. IR $\nu_{max}^{Neat}$ $cm^{-1}$: 3450 (OH), 1730 (OAc), 1610, 1580 (Ar) NMR $\delta_{ppm}^{CDCl_3}$: 1.1—1.8 [20H, m, $—(CH_2)_{10}—$], 2.02 (3H, s, OAc), 2.23 (3H, s, $C_5—CH_3$), 2.55 (2H, t, J=7Hz, $C_1—H_2$), 3.79 (3H, s, $OCH_3$), 3.83 (3H, s, $OCH_3$), 4.02 (2H, t, J=6Hz, $CH_2$OAc), 5.78 (1H, s, $C_1$—OH), 6.23 (1H, s, $C_4$—H) MS m/e: 394 ($M^+$), 352, 334, 181.

Potassium nitrosodisulphonate (18 g), water (300 ml) methanol (50 ml) and monopotassium phosphate (0.5 g) are added to a solution of 6-(12-acetoxydodecyl)-2,3-dimethoxy-5-methylphenol (6.1 g) in dimethylformamide (300 ml), and the mixture is stirred at room temperature for 30 days. The reaction mixture is subjected to extraction with dichloromethane and the organic layer is washed with water and dried over magnesium sulphate. The solvent is distilled off, thereby leaving crude crystals. The product is recrystallized from hexane to give 6-(12-acetoxydodecyl)-2,3-dimethoxy-5-methyl-1,4-benzoquinone (4.8 g) as orange-yellow needles. Melting point: 47°C.

Concentrated hydrochloric acid (0.1 ml) is added to a solution of 6-(12-acetoxydodecyl)-2,3-dimethoxy-5-methyl-1,4-benzoquinone (4.4 g) in methanol (200 ml), and the mixture is kept standing at room temperature for 12 hours. To the reaction mixture is added sodium hydrogen carbonate (0.2 g), and the solvent is distilled off. The residue is dissolved in dichloromethane and the insoluble materials are filtered off. Dichloromethane is distilled off, thereby leaving crude crystals. Recrystallization from hexane-ether (3:1) gives 6-(12-hydroxydodecyl)-2,3-dimethoxy-5-methyl-1,4-benzoquinone (3.8 g) as orange-yellow needles. Melting point: 63°C. IR$\nu_{max}^{KBr}$ $cm^{-1}$: 3550 (OH), 1650, 1640, 1610 (1-benzoquinone) NMR $\delta_{ppm}^{CDCl_3}$: 1.1—1.8 [20H, m, $—(CH_2)—10—$], 2.00 (3H, s, $C_5—CH_3$), 2.43 (2H, t, J=7Hz, $C_1—H_2$), 3.62 (2H, t, J=6Hz, $CH_2$OH), 397 (6H, s, $OCH_3$ X 2). MS m/e: 366 ($M^+$), 368 ($M^+ + 2$), 336, 197, 196, 195.

## Example 8

Potassium nitrosodisulphonate (30 g), water (1.5 1), methanol (300 ml) and monopotassium phosphate (1.3 g) are added to a solution of 6-(20-hydroxyeicosyl)-2,3-dimethoxy-5-methylphenol (2.3 g) in dimethylformamide (1.5 1), and the mixture is stirred at room temperature for 45 days. The reaction mixture is extracted with dichloromethane and recrystallized from ether. The above procedure gives 6-(20-hydroxyeicosyl)-2,3-dimethoxy-5-methyl-1,4-benzoquinone (1.3 g) as yellow needles. Melting point: 85°C. IR $\nu_{max}^{KBr}$ $cm^{-1}$: 3500 (OH), 1660, 1640, 1610 (1,4-benzoquinone) NMR $\delta_{ppm}^{CDCl_3}$: 1.1—1.8 [36H, m, $—(CH_2)_{18}—$], 2.00 (3H, s, $C_5—CH_3$), 2.43 (2H, t, J=7Hz, $C_1—H_2$), 3.62 (2H, t, J=6Hz, $—CH_2$OH), 3.96 (6H, s, $OCH_3$ X 2), MS m/e: 478 ($M^+$), 480 ($M^+ + 2$), 448, 450, 197, 196, 195.

## Example 9

A solution of potassium persulphate (2.7 g) in water (20 ml) is added to a solution of 6-(20-hydroxyeicosyl)-2,3-dimethoxy-5-methylphenol (0.6 g) in tetrahydrofuran, and the mixture is stirred in a nitrogen stream at room temperature for 72 hours. The reaction product is extracted with ether and ether is distilled off. To the residual 6-(20-hydroxyeicosyl)-2,3-dimethoxy-5-methylhydroquinone is added acetic anhydride (1 ml) and the mixture is kept standing at room temperature for 3 hours. The reaction mixture is extracted and treated in a conventional manner. The crude product thus obtained is recrystallized from hexane. The above procedure yields 6-(20-acetoxyeicosyl)-2,3-dimethoxy-5-methylhydroquinone-1,4-diacetate (0.31 g) as colourless needles. Melting point: 67°C.

By a similar manner to the corresponding procedure of Example 7, the product obtained above is treated with methanolic concentrated hydrochloric acid and then treated with methanolic ferric chloride, whereby 6-(20-hydroxyeicosyl)-2,3-dimethoxy-5-methyl-1,4-benzoquinone is obtained. m.p. 85°C.

## Example 10

Bis(salicylidene)ethylenediiminocobalt (II) (salcomine) (0.1 g) is added to a dimethylformamide solution (50 ml) of 6-(10-hydroxydecyl)-2,3-dimethoxy-5-methylphenol (2.53 g), and the mixture is stirred in an oxygen stream at atmospheric temperature and pressure for 72 hours. The solvent is then distilled off and the product is extracted with ether. The ethereal layer is washed with water and dried over anhydrous sodium sulphate, and the solvent is distilled off. The above procedure yields 6-(10-hydroxydecyl)-2,3-dimethoxy-5-methyl-1,4-benzoquinone (2.32 g) as orange-yellow needles metling at 52°C.

Example 11

Bis(3-methoxysalicylidene)ethylenediiminocobalt (II) 0.2 g) is added to a dimethylformamide solution (100 ml) of 6-(10-hydroxydecyl)-2,3-dimethoxy-5-methylphenol (4.1 g), and the mixture is stirred in an oxygen stream at atmospheric temperature and pressure for 72 hours. After the reaction has been completed, the solvent is distilled off and the residue is extracted with ether. The ethereal layer is washed with water and dried, and the solvent is distilled off in a conventional manner. The crude crystals thus obtained are recrystallized from ethyl acetate-hexane (1:3). The above procedure yields 6-(10-hydroxydecyl)-2,3-dimethoxy-5-methyl-1,4-benzoquinone (3.77 g) as orange-yellow needles melting at 52°C—53°C.

Example 12

Bis(salicylidene)ethylenediiminocobalt (II) (0.05 g) is added to a dimethylformamide solution (30 ml) of 6-(10-acetoxydecyl)-2,3-dimethoxy-5-methylphenol (1.6 g), and the mixture is stirred in an oxygen stream at atmospheric temperature and pressure for 72 hours. After the reaction has been completed, the solvent is distilled off and the product is isolated in the same manner as described in Example 10. The crude crystals thus obtained are recrystallized from hexane. The above procedure yields 6-(10-acetoxydecyl)-2,3-dimethoxy-5-methyl-1,4-benzoquinone (1.32 g) as orange-yellow needles melting at 38°C.

Example 13

Bis(salicylidene)ethylenediiminocobalt (II) (0.06 g) is added to a dichloromethane solution (30 ml) of 6-(10-hydroxy-decyl)-2,3-dimethoxy-5-methylphenol (1.4 g), and the mixture is stirred in an oxygen stream at 20°C and atmospheric pressure for 72 hours. After the reaction has been completed, the reaction mixture is passed through a bed of a small quantity of silica gel (about 15 g) to remove the catalyst. The solvent is then distilled off and the residue is recrystallized from ether-hexane (1:4). The above procedure yields 6-(10-hydroxydecyl)-2,3-dimethoxy-5-methyl-1,4-benzoquinone (1.01 g) as orange-yellow needles melting at 51—51°C.

Example 14

Bis-(3-methoxysalicylidene)ethylenediiminocobalt (II) (0.06 g) and pyridine (10 mg) are added to a dimethylformamide solution (30 mg) of 6-(10-hydroxy-decyl)-2,3-dimethoxy-5-methylphenol (1.6 g), and the mixture is stirred in an air stream at atmospheric temperature and pressure for 72 hours. After the reaction has been completed, the reaction product is isolated in the same manner as described in Example 10. The crude crystals thus obtained are recrystallized from ether-hexane (1:3). The above procedure yields 6-(10-hydroxydecyl)-2,3-dimethoxy-5-methyl-1,4-benzoquinone (1.1 g) as orange-yellow needles melting at 51°C—53°C.

Example 15

Pyridine (50 mg) and bis(4-hydroxysalicylidene)ethylenediiminocobalt (II) (36 mg) are added to a dimethylformamide solution (30 ml) of 6-(12-hydroxydodecyl)-2,3-dimethoxy-5-methylphenol (1.7 g), and the mixture is stirred in an oxygen stream at atmospheric temperature and pressure for 72 hours. The reaction product is isolated as in Example 10 and recrystallized from ether. The above procedure yields 6-(12-hydroxydodecyl)-2,3-dimethoxy-5-methyl-1,4-benzoquinone (1.37 g), m.p. 63°C.

Example 16

To a dimethylformamide solution (20 ml) of 6-(12-acetoxydodecyl)-2,3-dimethoxy-5-methylphenol (1.1 g) is added bis(salicylidene)ethylenediiminocobalt (II) (40 mg) and the mixture is stirred in an oxygen stream at atmospheric pressure and temperature for 72 hours. The product is isolated as in Example 10 and recrystallized from ether-hexane (1:1). The above procedure yields 6-(12-acetoxydodecyl)-2,3-dimethoxy-5-methyl-1,4-benzoquinone (0.84 g) as orange-yellow needles melting at 47°C.

Example 17

Bis(salicylidene)ethylenediiminocobalt (II) (40 mg) is added to a dimethylformamide solution (50 ml) of 6-(20-hydroxyeicosyl-2,3-dimethoxy-5-methylphenol (4.0 g), and the mixture is stirred in an oxygen stream at atmospheric temperature and pressure for 72 hours. The product is isolated as in Example 10 and recrystallized from ether. The above procedure yields 6-(20-hydroxyeicosyl)-2,3-dimethoxy-5-methyl-1,4-benzoquinone (3.28 g) as yellow needles melting at 85°C.

Example 18

Bis(salicylidene)ethylenediiminocobalt (II) (10 mg) and sodium cyanide (2 mg) are added to a dimethylformamide solution (10 ml) of 1-acetoxy-6-(10-acetoxydecyl)-2,3-dimethoxy-5-methylbenzene (179 mg), followed by stirring in an oxygen stream at atmospheric temperature and pressure for 96 hours. After the reaction has been completed, 10 to 30 mg of ferric chloride are added to make the sodium cyanide-iron complex and, thereafter, the desired compound is isolated in the same manner as in Example 10. Recrystallization of the product from hexane yields 6-(10-acetoxydecyl)-2,3-dimethoxy-5-methyl-1,4-benzoquinone (103 mg) as orange-yellow needles melting at 37°C—38°C.

Example 19

Bis(salicylidene)ethylenediiminocobalt (II) (14 mg) is added to a dimethylformamide solution (20 ml) of 6-(10-acetoxydecyl)-2,3-dimethoxy-5-methyl-1-tetrahydropyranyloxybenzene (240 mg), followed by stirring in an oxygen stream at atmospheric temperature and pressure for 72 hours. The reaction product is isolated in the same manner as in Example 10 and recrystallized from hexane. The above procedure yields 6-(10-acetoxydecyl)-2,3-dimethoxy-5-methyl-1,4-benzoquinone (110 mg) as orange-yellow needles melting at 37°C—38°C.

Example 20

Bis(salicylidene)ethylenediiminocobalt (II) (10 mg) is added to a dimethylformamide solution of 10-(3,4-dimethoxy-2-hydroxy-6-methylphenyl)decanoic acid (100 mg), followed by stirring in an oxygen stream at atmospheric temperature and pressure for 72 hours. The desired compound is isolated in the same manner as in Example 10 and recrystallized from ether. The above procedure yields 6-(9-carboxynonyl)-2,3-dimethoxy-5-methyl-1,4-benzoquinone (78 mg) as orange-yellow needles melting at 59°C—60°C.

Example 21

A dimethylformamide solution of methyl 10-(3,4-dimethoxy-2-hydroxy-6-methylphenyl)-decanoate (129 mg) is oxidized in the same manner as in Example 20. This procedure yields 2,3-dimethoxy-5-methyl-6-(9-methoxycarbonylnonyl)-1,4-benzoquinone (91 mg) as orange-yellow needles melting at 37°C—38°C.

Example 22

10-(2-Hydroxy-3,4,6-trimethylphenyl)decanoic acid (270 mg) is oxidized in the same manner as in Example 10. This procedure yields 2,3,5-trimethyl-6-(9-carboxynonyl)-1,4-benzoquinone (200 mg) as an orange-yellow oil. IR spectrum $\nu_{max}^{Neat}$ $cm^{-1}$: 1705 (COOH), 1640, 1610 (quinone).

**Claims**

1. A compound of the formula:

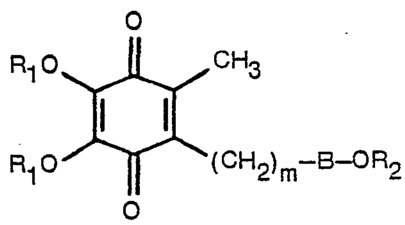

wherein $m$ is an integer of 10 to 21; $R_1$ is lower alkyl having 1 to 4 carbon atoms; B is a $—CH_2—$ or —CO— group; when B is $—CH_2—$, $R_2$ is hydrogen, lower alkyl having 1 to 4 carbon atoms or lower acyl having 1 to 8 carbon atoms, and when B is —CO—, $R_2$ is hydrogen or lower alkyl having 1 to 4 carbon atoms; provided that, where $R_1$ is methyl, B is $—CH_2—$ and $R_2$ is hydrogen, then $m$ is 11 or 19 only, and excluding the following compounds:
2,3-dimethoxy-6-(12-methoxycarbonyldodecyl)-5-methyl-1,4-benzoquinone, and
6-(11-acetoxyundecyl)-2,3-dimethoxy-5-methyl-1,4-benzoquinone.

2. A compound as claimed in claim 1, wherein B is a $—CH_2—$ group.
3. A compound as claimed in claim 1, wherein B is a —CO— group.
4. A compound as claimed in claim 1, wherein $R_2$ is hydrogen.
5. A compound as claimed in claim 1, wherein $R_2$ is lower acyl having 1 to 8 carbon atoms.
6. A compound as claimed in claim 1, wherein $R_1$ is methyl.
7. A compound as claimed in claim 1, wherein $m$ is an integer of 10 to 17.
8. A method for producing a compound of the formula:

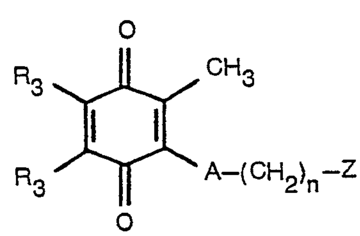

(II)

[wherein each $R_3$ is lower alkyl having 1 to 4 carbon atoms or lower alkoxy having 1 to 4 carbon atoms, or two $R_3$'s taken together represent —CH=CH—CH=CH—; *n* is 0 or an integer of 8 to 20 and when *n* is 8 to 20, A is $—CH_2—$,

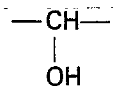

or —CO— group and Z is $CH_2CH$, acyloxymethyl having 2 to 11 carbon atoms esterified- or unesterified carboxyl having 1 to 5 carbon atoms, lower alkoxy methyl having 2 to 5 carbon atoms, silyloxymethyl having 4 to 7 carbon atoms or acetalmethyl having 3 to 8 carbon atoms; when *n* is 0, $—A—(CH_2)_n—Z$ is

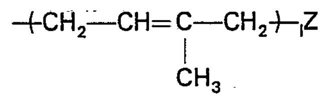

(wherein *l* is an integer of 3 to 12) and Z is hydrogen], which comprises oxidizing a compound of the formula:

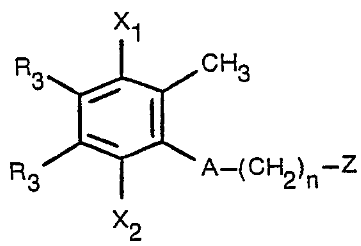

(III)

(wherein either $X_1$ or $X_2$ is hydroxyl or amino, with the other being hydrogen or an esterified hydroxyl group having 1 to 8 carbon atoms or etherified hydroxyl group having 2 to 8 carbon atoms; the other symbols are as defined above) with oxygen or air in the presence of a cobalt-complex compound of the formula:

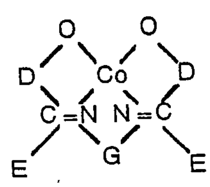

(IV)

[wherein D is a vinylene group of

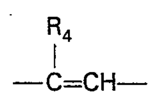

($R_4$ is lower alkyl or phenyl) or an orthophenylene group of

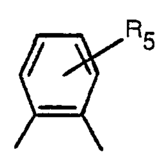

($R_5$ is H, hydroxy or lower alkoxy); E is H or lower alkyl; G is an ethylene group of

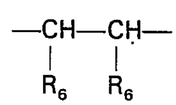

($R_6$ is H or lower alkyl) or an orthophenylene group of

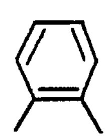 ],

but excluding the production of 6-(22-hydroxydocosyl)-2,3-dimethoxy-5-methyl-1,4-benzoquinone by oxidation of 6-(22-hydroxydocosyl)-2,3-dimethoxy-5-methyl phenol in the presence of bis(4-hydroxysalicylidene)ethylenediimino-cobalt (II).

## Revendications

1. Composé de formule

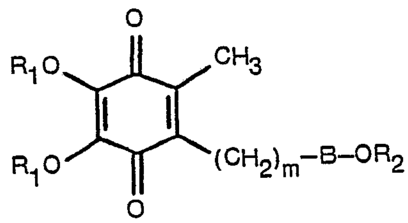

dans laquelle *m* est un nombre entier de 10 à 21; $R_1$ est un radical alkyle inférieur ayant 1 à 4 atomes de carbone; B est un groupe —$CH_2$— ou —CO—; quand B est —$CH_2$—, $R_2$ représente un atome d'hydrogène, un radical alkyle inférieur ayant 1 à 4 atomes de carbone ou acyle inférieur ayant 1 à 8 atomes de carbone, et quand B est —CO—, $R_2$ représente un atome d'hydrogène ou un radical alkyle inférieur ayant 1 à 4 atomes de carbone; étant entendu que si $R_1$ est un radical méthyle, si B est —$CH_2$— et que $R_2$ est un atome d'hydrogène, alors *m* ne vaut que 11 ou 19, et à l'exclusion des composés suivants:
2,3-diméthoxy-6-(12-méthoxycarbonyldodécyl)-5-méthyl-1,4-benzoquinone, et
6-(11-acétoxyundécyl)-2,3-diméthoxy-5-méthyl-1,4-benzoquinone.

2. Composé selon la revendication 1, dans lequel B est un groupe —$CH_2$—.
3. Composé selon la revendication 1, dans lequel B est un groupe —CO—.
4. Composé selon la revendication 1, dans lequel $R_2$ est un atome d'hydrogène.
5. Composé selon la revendication 1, dans lequel $R_2$ est un radical acyle inférieur ayant 1 à 8 atomes de carbone.
6. Composé selon la revendication 1, dans lequel $R_1$ est le radical méthyle.
7. Composé selon la revendication 1, dans lequel *m* est un nombre entier de 10 à 17.
8. Procédé de préparation d'un composé de formule:

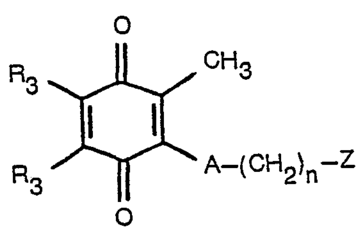

(II)

[dans laquelle chaque $R_3$ représente un radical alkyle inférieur ayant 1 à 4 atomes de carbone ou alcoxy inférieur ayant 1 à 4 atomes de carbone, ou bien deux $R_3$ pris ensemble représentent le groupe —CH=CH—CH=CH—; *n* est 0 ou un nombre entier de 8 à 20, et lorsque *n* est 8 à 20, A représente un groupe —$CH_2$—,

—CH—
|
OH

ou —CO—, et Z est un groupe $CH_2OH$, acyloxyméthyle ayant de 2 à 11 atomes de carbone, carboxyle estérifié ou non estérifié ayant 1 à 5 atomes de carbone, alcoxymèthyle inférieur ayant de 2 à 5 atomes de carbone, silyloxyméthyle ayant 4 â 6 atomes de carbone, ou acétalméthyle ayant 3 à 8 atomes de carbone; quand *n* est 0, —A—$(CH_2)_n$—Z est

$-(CH_2-CH=C-CH_2)_l-Z$
|
$CH_3$

(l étant un nombre entier de 3 à 12), et Z est un atome d'hydrogène], qui consiste à oxyder un composé de formule:

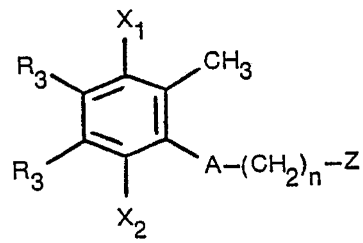

(III)

(dans laquelle $X_1$ ou $X_2$ représente un radical hydroxyle ou amino alors que l'autre est un atome d'hydrogène ou un groupe hydroxyle estérifié ayant 1 à 8 atomes de carbone ou un groupe hydroxyle éthérifié ayant 2 à 8 atomes de carbone; les autres symboles sont comme définis plus haut) avec de l'oxygène ou de l'air en présence d'un composé complexe de cobalt de formule:

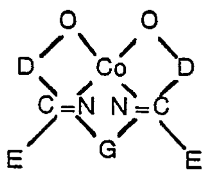

(IV)

[dans laquelle D est un groupe vinylène de

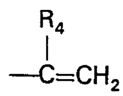

($R_4$ est un radical alkyle inférieur ou phényle) ou un groupe orthophényle de

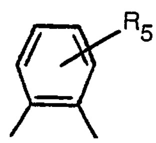

($R_5$ représente H, ou un groupe hydroxyle ou alcoxy inférieur); E représente H ou un radical alkyle inférieur; G est un groupe éthylène

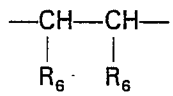

($R_6$ est H ou un radical alkyle inférieur] ou un groupe orthophénylène de

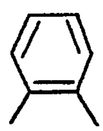

],

mais à l'exclusion de la préparation de 6-(22-hydroxydocosyl)-2,3-diméthoxy-5-méthyl-1,4-benzoquinone par oxydation de 6-(22-hydroxydocosyl)-2,3-diméthoxy-5-méthylphénol en présence de bis (4-hydroxysalicylidène)éthylènedi-imino-cobalt (II).

**Patentansprüche**

1. Eine Verbindung der Formel

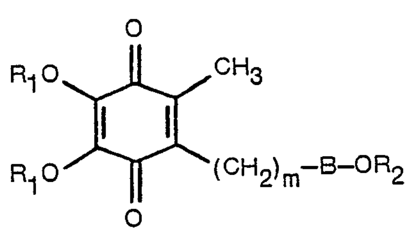

worin m eine ganze Zahl von 10 bis 21, $R_1$ ein neiderer Alkylrest mit 1 bis 4 Kohlenstoffatomen, B eine $—CH_2—$ oder —CO-Gruppe ist; wenn B für $—CH_2—$ steht, $R_2$ Wasserstoff, ein niederer Alkylrest mit 1 bis 4 Kohlenstoffatomen oder ein niederer Acylrest mit 1 bis 8 Kohlenstoffatomen ist und, wenn B für —CO— steht, $R_2$ Wasserstoff oder ein niederer Alkylrest mit 1 bis 4 Kohlenstoffatomen ist mit der Maßgabe, daß, wenn $R_1$ Methyl ist, B für $—CH_2—$ und $R_2$ für Wasserstoff steht, dann m nur 11 oder 19 ist, und ausschließlich der folgenden Verbindungen:
2,3-Dimethoxy-6-(12-methoxycarbonyldodecyl)-5-methyl-1,4-benzochinon und
6-(11-Acetoxyundecyl)-2,3-dimethoxy-5-methyl-1,4-benzochinon.

2. Eine Verbindung nach Anspruch 1, worin B eine $—CH_2—$ Gruppe ist.

3. Eine Verbindung nach Anspruch 1, worin B eine —CO— Gruppe ist.

4. Eine Verbindung nach Anspruch 1, worin $R_2$ Wasserstoff ist.

5. Eine Verbindung nach Anspruch 1, worin $R_2$ ein niederer Acylrest mit 1 bis 8 Kohlenstoffatomen ist.

6. Eine Verbindung nach Anspruch 1, worin $R_1$ Methyl ist.

7. Eine Verbindung nach Anspruch 1, worin m eine ganze Zahl von 10 bis 17 ist.

8. Verfahren zur Herstellung einer Verbindung der Formel

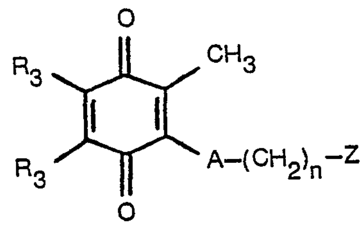

(II)

[worin jeder Rest $R_3$ ein niederer Alkylrest mit 1 bis 4 Kohlenstoffatomen oder ein niederer Alkoxyrest mit 1 bis 4 Kohlenstoffatomen ist oder zwei Reste $R_3$ gemeinsam —CH=CH—CH=CH— darstellen; n für 0 oder eine ganze Zahl von 8 bis 20 steht und, wenn n für 8 bis 20 steht, A für $—CH_2$, eine

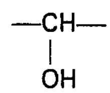

oder —CO—Gruppe und Z für $CH_2OH$, Acyloxymethyl mit 2 bis 11 Kohlenstoffatomen, verestertes oder nicht verestertes Carboxyl mit 1 bis 5 Kohlenstoffatomen, niederes Alkoxymethyl mit 2 bis 5 Kohlenstoffatomen, Silyloxymethyl mit 4 bis 7 Kohlenstoffatomen oder Acetalmethyl mit 3 bis 8 Kohlenstoffatomen steht; wenn n für 0 steht, $—A—(CH_2)_n—Z$ für

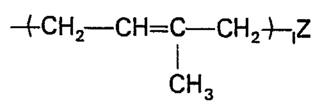

(worin 1 eine ganze Zahl von 3 bis 12 ist) und Z für Wasserstoff steht], dadurch gekennzeichnet, daß man eine Verbindung der Formel

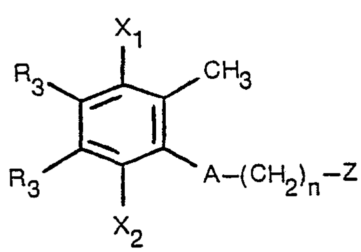

(III)

(worin entweder $X_1$ oder $X_2$ Hydroxyl oder Amino und der andere Rest Wasserstoff oder eine veresterte Hydroxylgruppe mit 1 bis 8 Kohlenstoffatomen oder eine veretherte Hydroxylgruppe mit 2 bis 8 Kohlenstoffatomen ist, während die anderen Symbole die vorstehend genannten Bedeutungen haben) mit Sauerstoff oder Luft in Gegenwart eine Kobaltkomplexverbindung der Formel

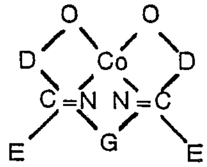

(IV)

[worin D eine Vinylengruppe

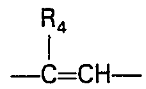

(worin $R_4$ ein niederer Alkylrest oder ein Phenylrest ist) oder eine o-Phenylengruppe

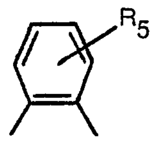

($R_5$ ist H, Hydroxy oder niederes Alkoxy) E für H oder niederes Alkyl, G für eine Ethylengruppe

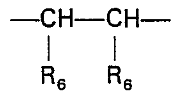

($R_6$ ist H oder niederes Alkyl) oder eine o-Phenylengruppe

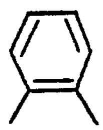

steht] oxidiert, jedoch ausschließlich der Bildung von 6-(22-Hydroxydocosyl)-2,3-dimethoxy-5-methyl-1,4-benzochinon durch Oxidation von 6-(22-Hydroxydocosyl)-2,3-dimethoxy-5-methylphenol in Gegenwart von Bis(4-hydroxysalicyliden)ethylendiiminocobalt (II).